# EUROPEAN PATENT SPECIFICATION

(11) **EP 2 921 107 B1**
(45) Date of publication and mention of the grant of the patent: **07.09.2022**
(21) Application number: 14189721.5
(22) Date of filing: 21.10.2014
(51) Int. Cl.: A61B 5/00

(54) **Parallel signal processor with amplifiers and with converters and a method for its operation**
Paralleler Signalprozessor mit Verstärkern und mit Umformern und ein Verfahren zu dessen Betrieb
Processeur de signaux en parallèle avec des amplificateurs et avec des convertisseurs et un procédé pour son opération

(30) Priority: 12.03.2014 KR 20140029067
(43) Date of publication of application: 23.09.2015
(73) Proprietor: Samsung Electronics Co., Ltd., Gyeonggi-do 443-742 (KR)
(72) Inventor: Bae, Chi Sung, 443-803 Gyeonggi-do (KR); Kim, Sang Joon, 443-803 Gyeonggi-do (KR); Choi, Chang Mok, 443-803 Gyeonggi-do (KR)
(74) Representative: Grünecker Patent- und Rechtsanwälte PartG mbB

(56) References cited:
- US-A1- 2009 247 894
- US-A1- 2011 213 265
- US-A1- 2012 150 059
- US-A1- 2013 053 722

## Description

### 1. Field

The following description relates to a parallel biometric signal processor and a method of controlling the parallel biometric signal processor.

### 2. Description of Related Art

Various medical apparatuses are being developed to diagnose health conditions of a patient. As interest in ubiquitous healthcare or U-health increases, new technologies are being developed to monitor and analyze vital signs in daily life. For example, extensive research is being conducted to develop a biometric signal processor that continuously monitors biological conditions and reactions of a user.

The biometric signal processor would be required to be ultra-light and extra-small for user convenience. Accordingly, the biometric signal processor would also need to be of low power consumption within a capacity of a light weight and small battery.

US 2011/213265 discloses a control module for connecting a plurality of brainwave detecting electrodes, the module comprising an electrode selector for selecting which electrodes should be connected to a sensing channel, a sensing front end performing amplification and analog-to-digital conversion, a wave morphology analysis unit for extracting feature information from an input waveform and an event detector logic.

### SUMMARY

This Summary is provided to introduce a selection of concepts in a simplified form that are further described below in the Detailed Description.

In accordance with an illustrative example, there is provided a parallel biometric signal processor, including amplifiers configured to amplify a biometric signal based on an amplifying attribute; converters configured to convert the amplified signal to a converted signal based on a converting attribute; preprocessors configured to preprocess the converted signal based on a preprocessing attribute; and feature extractors configured to extract a set of biometric information from an output signal of the preprocessors.

Switching fabrics are connected among the amplifiers, the converters, the preprocessors, and the feature extractors.

The processor includes a switching controller configured to perform routing on a target amplifier of the amplifiers to which the biometric signal is input, a target converter of the converters to which the amplified signal of the target amplifier is input, a target preprocessor to which the converted signal of the target converter is input, and a target feature extractor to which the output signal of the target preprocessor is input, by controlling the switching fabrics.

The switching controller is configured to perform rerouting through the target amplifier, the target converter, the target preprocessor, and the target feature extractor.

The switching controller is configured to perform the routing through the target amplifier, the target converter, the target preprocessor, and the target feature extractor based on an operating mode.

The operating mode may include one of a low power operating mode and a high precision operating mode.

The amplifiers, the converters, the preprocessors, and the feature extractors may be connected in parallel.

The amplifiers may possess different amplified attributes, wherein the converters includes different converting attributes, wherein the preprocessors includes different preprocessing attributes, and wherein the feature extractors are configured to extract different sets of the biometric information.

The processor includes ports connected to at least one sensor configured to sense the at least one biometric signal.

The processor includes a switching fabric disposed between the ports and the amplifiers, wherein the switching controller is configured to provide the biometric signal to the target amplifier by controlling the switching fabric disposed between the ports and the amplifiers.

The sensor may include at least one of an electrode sensor, a photochemical sensor, and a photoelectric sensor.

The amplified attribute may include at least one of an input impedance, a bandwidth, and an amplification gain.

The amplifiers may be configured to adjust the amplified attribute.

The amplifiers may include at least one of an instrument amplifier (IA), a programmable gain amplifier (PGA), and a band pass filter (BPF).

The converters may be configured to convert the amplified signal to a digital signal based on the converting attribute.

The converting attribute may include at least one of an input dynamic range and an output bit resolution.

The preprocessing attribute may include at least one of an attribute of filtering an unnecessary frequency band of the converted signal and an attribute of extracting a set of preprocessing information from the converted signal.

The at least one set of preprocessing information may include at least one of information on a time at which the converted signal is acquired and information on a frequency characteristic of the converted signal.

The processor may also include a power controller configured to control power to be provided to the amplifiers, the converters, the preprocessors, and the feature extractors.

The processor may also include a register controller configured to control detailed attributes of the amplifiers, the converters, the preprocessors, and the feature extractors.

The processor may include a transmitter configured to transmit the biometric information to an external device.

The processor may include an interface wiredly connected to the external device, wherein the transmitter is configured to transmit the biometric information to the external device using the interface.

The interface may include at least one of a universal asynchronous receiver transmitter (UART), a serial peripheral interface (SPI), and an inter-integrated circuit (I2C).

The processor may also include an interface wirelessly connected to the external device, wherein the transmitter is configured to transmit the biometric information to the external device using the interface.

The interface may include at least one of body area network (BAN), Bluetooth, ZigBee, and near field communication (NFC).

In accordance with an example, there is provided an application processor according to claim 1, including a processor core configured to process commands and data; and a parallel biometric signal processor configured to extract a set of biometric information from a biometric signal, wherein the parallel biometric signal processor includes amplifiers configured to amplify the biometric signal into at an amplified attribute, converters configured to convert an amplifying signal of the amplifiers to a converting attribute, preprocessors configured to preprocess a converted signal of the converters based on a preprocessing attribute, feature extractors configured to extract the set of the biometric information from an output signal of the preprocessors, and switching fabrics connected among the amplifiers, the converters, the preprocessors, and the feature extractors.

In accordance with an illustrative example, there is provided a method of controlling a parallel biometric signal processor according to claim 11, including performing routing through amplifiers, converters, preprocessors, and feature extractors, amplifying a biometric signal through a target amplifier of the amplifiers; converting the amplified biometric signal through a target converter of the converters; preprocessing the converted signal through a target preprocessor of the preprocessors; and extracting a set of biometric information from the preprocessed signal through a feature extractor of the feature extractors .

In accordance with an illustrative example, there is provided a parallel biometric signal processor according to claim 1, including a controller configured to receive a first and a second signals, and simultaneously process the first and the second signals by simultaneously routing the first signal through a first path and the second signal through a second path, wherein through the first path, the controller converts the first signal to a first converted signal using a first converting attribute, preprocesses the first converted signal based on a first preprocessing attribute, and extracts first biometric information, and through the second path, the controller converts the second signal to a second converted signal using a second converting attribute, preprocesses the second converted signal based on a second preprocessing attribute, and extracts second biometric information.

The controller may be further configured to amplify the first signal using a first amplifying attribute and the second signal using a second amplifying attribute prior to converting the first and the second signals.

The first and the second amplifying attributes may include at least one of an input impedance, a bandwidth, and an amplification gain, the first and the second converting attributes include at least one of an input dynamic range and an output bit resolution, and the first and the second preprocessing attributes include at least one of an attribute of filtering an unnecessary frequency band and an attribute of extracting at least one set of preprocessing information.

The controller includes amplifiers to amplify the first and the second signals, converters to convert the amplified first signal to the first converted signal and to convert the amplified second signal to the second converted signal, preprocessors to preprocess the first converted signal and the second converted signal, and feature extractors to extract the first and the second biometric information.

Ports are connected to at least one sensor configured to sense the at least one biometric signal.

The processor includes a port switching fabric disposed between the ports and the amplifiers, an amplifying switching fabric disposed between the amplifiers and the converters, a converter switching fabric disposed between the converters and the preprocessors, and a preprocessor switching fabric disposed between the preprocessors and the feature extractors.

Other features and aspects will be apparent from the following detailed description, the drawings, and the claims.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating an example of a parallel biometric signal processor.
FIG. 2 is a diagram illustrating an example of the parallel biometric signal processor.
FIGS. 3 through 5 are diagrams illustrating examples of operations of the parallel biometric signal processor.
FIG. 6 is a block diagram illustrating an example of an integrated biometric signal processor.
FIG. 7 is a block diagram illustrating an example of an application processor.
FIG. 8 is a flowchart illustrating an example of a method to control the parallel biometric signal processor.

Throughout the drawings and the detailed description, unless otherwise described or provided, the same drawing reference numerals will be understood to refer to the same elements, features, and structures. The drawings may not be to scale, and the relative size, proportions, and depiction of elements in the drawings may be exaggerated for clarity, illustration, and convenience.

### DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art. Also, descriptions of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

It will be understood that when an element or layer is referred to as being "on" or "connected to" another element or layer, it can be directly on or connected to the other element or layer or through intervening elements or layers may be present. In contrast, when an element is referred to as being "directly on" or "directly connected to" another element or layer, there are no intervening elements or layers present. Like reference numerals refer to like elements throughout.

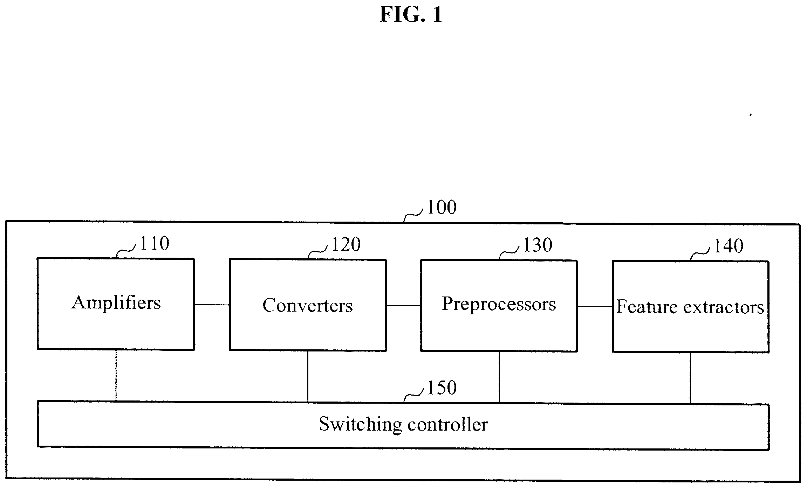

FIG. 1 is a block diagram illustrating an example of a parallel biometric signal processor 100.

Referring to FIG. 1, the parallel biometric signal processor 100 includes amplifiers 110, converters 120, preprocessors 130, feature extractors 140, and a switching controller 150.

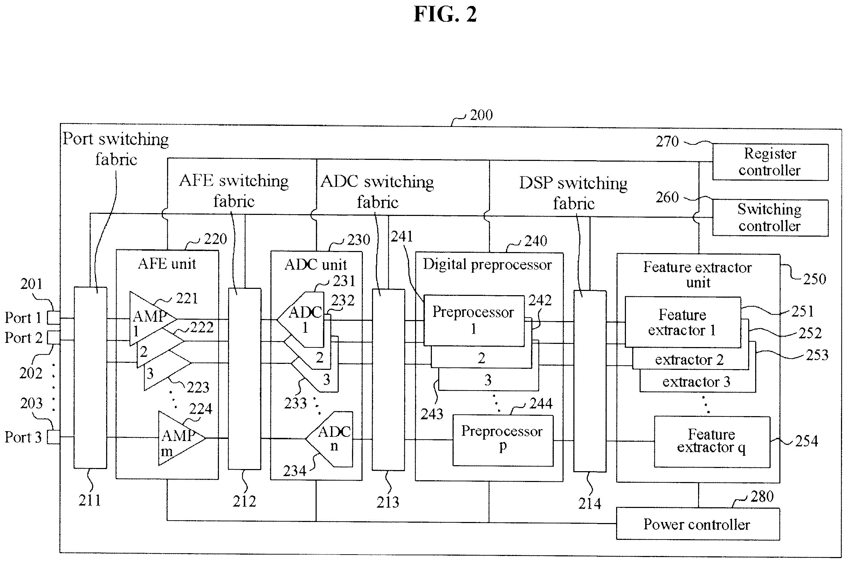

The parallel biometric signal processor 100 further includes a register controller, a power controller, switching fabrics, ports, and a transmitter, which will be described hereinafter with reference to FIG. 2.

In one illustrative example, the parallel biometric signal processor 100 is configured in a form of a system on chip (SoC).

The amplifiers 110 are arranged in parallel and amplify at least one biometric signal based on at least one amplifying attribute. In one example, at least one signal to be amplified by the amplifiers 110 is an analog signal. Due to the parallel arrangement of the amplifiers 110, each of the amplifiers 110 may amplify a biometric signal based on a predetermined amplifying attribute, independently from other amplifiers 110 and without adversely affecting the performance of the other amplifiers 110. The amplifying attribute may include at least one of an input impedance, a bandwidth, and an amplification gain. The amplifiers 110 may have different amplifying attributes or adaptively adjust the amplifying attribute. Alternatively, the amplifying attribute may be adjusted by a register controller, illustrated in FIG. 2, to be described hereinafter. Also, the amplifiers 110 may include at least one of an instrument amplifier (IA), a programmable gain amplifier (PGA), and a band pass filter (BPF). Although in one configuration, the amplifiers 110 are included within the parallel biometric signal processor 100, in another configuration, the amplifiers 110 may be external to the parallel biometric signal processor 100, while still being controlled by the register controller.

The converters 120 are arranged in parallel and convert an amplifying signal from the amplifiers 110 to a converted signal based on at least one converting attribute. In an example, the converters 120 convert an amplifying signal of the amplifiers 110 to a digital signal based on the converting attribute. In one configuration, each converter 120 possesses different converting attributes. As a result of the parallel arrangement of the converters 120, each of the converters 120 may convert an analog signal to a digital signal based on a predetermined converting attribute, independently from other converters 120 and without adversely affecting the performance of the other converters 120. The converting attribute may include at least one of an input dynamic range and an output bit resolution.

The preprocessors 130 are arranged in parallel and preprocess a converted signal from the converters 120 based on at least one preprocessing attribute. The preprocessing attribute is an operation of signal processing to be performed in advance to enable the feature extractors 140 to extract biometric information. In one illustrative example, at least one of the preprocessors 130 possesses a different preprocessing attribute from the preprocessors 130. Due to the parallel arrangement of the preprocessors 130, each of the preprocessors 130 preprocesses the converted signal based on a predetermined preprocessing attribute, independently from other preprocessors 130 and without adversely affecting the performance of the other preprocessors 130. The preprocessing attribute may include at least one of an attribute of filtering an unnecessary frequency band of the converted signal and an attribute of extracting at least one set of preprocessing information from the converted signal. Also, the preprocessing information includes at least one of information about a time at which the converted signal is acquired and information about a frequency characteristic of the converted signal. The preprocessors 130 may be configured as a fixed hardware block in a micro controller unit (MCU).

The feature extractors 140 are arranged in parallel and extract at least one set of biometric information from an output signal of the preprocessors 130. The biometric information may include information measured from a body of a user such as a blood glucose level, blood pressure, weight, and an electrocardiogram (ECG), information generated by a movement of the user such as a number of strides, and medical information in association with, for example, arrhythmia, angina, and myocardial infarction (MI). In one illustrative example, the feature extractors 140 extract heart rate information by discovering R-peak information from a preprocessed ECG signal and extract the information from the heart rates by discovering peak information from a preprocessed photoplethysmogram (PPG) signal. In another example, the feature extractors 140 extract blood pressure information indicative of a difference in peaks of the preprocessed ECG signal and the preprocessed PPG signal, extract information of a movement of a muscle from a preprocessed electromyogram (EMG) signal, and extract information of a brain activity from an electroencephalogram (EEG) signal. The feature extractors 140 may be configured as a fixed hardware block in an MCU.

Ports (not shown) are connected to at least one sensor (not shown) that senses at least one biometric signal. The ports receive the at least one biometric signal from the sensor and transmit the received biometric signal to the amplifiers 110. The sensor may include an electrode sensor to measure a difference of electrical potentials in human body portions, a photochemical sensor, such as, an ion sensitive field effect transistor (ISFET), and a photoelectric sensor, for example, a photodiode sensor, to detect emission, absorption, fluorescence, and reflection of a light.

Switching fabrics (not shown) interconnect the amplifiers 110, the converters 120, the preprocessors 130, and the feature extractors 140. Also, a switching fabric is disposed between the ports and the amplifiers 110. A second switching fabric is disposed between the amplifiers 110 and the converters 120. A third switching fabric is disposed between the converters 120 and the preprocessors 130. A fourth switching fabric is disposed between the preprocessors 130 and the feature extractors 140.

The switching controller 150 controls the switching fabrics and performs routing on at least one target amplifier to which a biometric signal is input, at least one target converter to which an amplifying signal of the target amplifier is input, at least one target preprocessor to which a converted signal of the target converter is input, and at least one feature extractor to which an output signal of the preprocessor is input. The target amplifier, the target converter, the target preprocessor, and the target feature extractor respectively refer to one of the amplifiers 110, the converters 120, the preprocessors 130, and the feature extractors 140 that may be used to extract applicable biometric information.

The switching controller 150 selects an amplifier, a converter, a preprocessor, and a feature extractor to extract the biometric information from the amplifiers 110, the converters 120, the preprocessors 130, and the feature extractors 140, respectively. Accordingly, accuracy of the biometric information to be extracted is improved, a degree of freedom and an efficiency of the parallel biometric signal processor 100 is improved, and desired biometric information is extracted based, in one example, solely the parallel biometric signal processor 100, without assistance from another processor.

The switching controller 150 performs the routing on a target port that forwards a biometric signal received from the sensor to the target amplifier. In addition, the switching controller 150 also forwards the biometric signal from the target port to the target amplifier, the target converter, the target preprocessor, and the target feature extractor. The target port refers to a port to be used to extract applicable biometric information among the ports.

To extract desired information from the at least one biometric signal, the switching controller 150 connects a target amplifier, a target converter, a target preprocessor, and a target feature extractor that may meet required specifications. For example, to extract information on heart rates from an electrocardiography (ECG) signal, the switching controller 150 performs routing on a target amplifier having an amplification gain of 100 among the amplifiers 110, a target converter having a converting attribute of a 12 bit resolution among the converters 120, a target preprocessor having a preprocessing attribute of eliminating a direct current (DC) offset among the preprocessors 130, and a target feature extractor extracting a heart rate among the feature extractors 140. Through the routing by the switching controller 150, the target amplifier amplifies by a factor of one hundred, the ECG signal having a frequency band in a range 0.5 hertz (Hz) to 40 Hz. The target converter converts an amplifying signal to a digital signal based on the 12 bit resolution. The target preprocessor eliminates the DC offset of a converted signal, and the target feature extractor extracts the information on the heart rates by discovering an R peak from the preprocessed ECG signal.

The switching controller 150 performs the routing of the at least one biometric signal through the target amplifier, the target converter, the target preprocessor, and the target feature extractor based on routing paths. For example, when each of a number of the amplifiers 110, the converters 120, the preprocessors 130, and the feature extractors 140 is three, a first routing path used to extract the information on the heart rates from the ECG signal includes a first amplifier, a first converter, a third preprocessor, and a third feature extractor. A second routing path used to extract the information on the movement of the muscle from the EMG signal includes a second amplifier, a second converter, a first preprocessor, and a second feature extractor. Based on the first routing path and the second routing path, the switching controller 150 simultaneously processes the ECG signal and the EMG signal by simultaneously performing the routing through the first and second target amplifiers, the first and second target converters, the third and first target preprocessors, and the third and second target feature extractors. The term simultaneously may be defined as a same time processing the ECG signal and the EMG signal or one signal after another within a minimal time frame difference or within a small or negligible margin of time difference.

The switching controller 150 reroutes a signal through the target amplifier, the target converter, the target preprocessor, and the target feature extractor. For example, when each of a number of the amplifiers 110, the converters 120, the preprocessors 130, and the feature extractors 140 is four, and the blood pressure information to be extracted is from a biometric signal, the switching controller 150 selects the first amplifier, a third converter, the third preprocessor, and a fourth feature extractor as the target amplifier, the target converter, the target preprocessor, and the target feature extractor, respectively. When the information on the movement of the muscle is to be extracted from the biometric signal, the switching controller 150 selects the second amplifier, the first converter, a second preprocessor, and the third feature extractor as the target amplifier, the target converter, the target preprocessor, and the target feature extractor, respectively.

Also, the switching controller 150 performs the routing through the target amplifier, the target converter, the target preprocessor, and the target feature extractor based on an operating mode. The operating mode may include a low power operating mode and a high precision operating mode. For example, when each of a number of the amplifiers 110, the converters 120, the preprocessors 130, and the feature extractors 140 is two, respectively, a first amplifier, a first converter, and a first preprocessor has a lower performance and less power consumption than a second amplifier, a second converter, and a second preprocessor. A first feature extractor extracts information of an arrhythmia, while a second feature extractor extracts information on brain activity. When the operating mode is the low power operating mode, the switching controller 150 selects the first amplifier, the first converter, the first preprocessor, and the first feature extractor as the target amplifier, the target converter, the target preprocessor, and the target feature extractor, respectively. Conversely, when the operating mode is the high precision operating mode, the switching controller 150 selects the second amplifier, the second converter, the second preprocessor, and the first feature extractor as the target amplifier, the target converter, the target preprocessor, and the target feature extractor, respectively.

The register controller (to be later discussed with respect to FIG. 2) monitors and controls detailed attributes of the amplifiers 110, the converters 120, the preprocessors 130, and the feature extractors 140. For example, the register controller adjusts an input impedance, a bandwidth, and an amplification gain of the amplifiers 110, an input dynamic range and an output bit resolution of the converters 120, and bandwidths and sampling rates of the preprocessors 130 and the feature extractors 140, based on a control signal from the switching controller 150 or an external source.

The power controller (to be later discussed with respect to FIG. 2) controls power to be provided to the amplifiers 110, the converters 120, the preprocessors 130, the feature extractors 140, and the ports. For example, the power controller provides the power to the target port, the target amplifier, the target converter, the target preprocessor, and the target feature extractor on which the routing is performed by the switching controller 150. The power controller also blocks the power to remaining ports, remaining amplifiers, remaining converters, remaining preprocessors, and remaining feature extractors. Accordingly, the parallel biometric signal processor 100 reduces power consumption.

A transmitter transmits the biometric information extracted by the target feature extractor to an external device. In one example, the transmitter includes at least one of a wired interface to be wiredly connected to the external device and a wireless interface to be wirelessly connected to the external device. The transmitter transmits the biometric information to the external device using the wired interface or the wireless interface. The wired interface includes at least one of a universal asynchronous receiver transmitter (UART), a serial peripheral interface (SPI), and an inter-integrated circuit (I2C). The wireless interface includes at least one of body area network (BAN), Bluetooth, ZigBee, and near field communication (NFC).

FIG. 2 is a diagram illustrating an example of a parallel biometric signal processor 200.

Referring to FIG. 2, the parallel biometric signal processor 200 includes 1 to k ports, for example, 201 through 203, an analog front end (AFE) unit 220, an analog to digital converter (ADC) unit 230, a digital preprocessor 240, a feature extractor 250, a switching controller 260, a register controller 270, and a power controller 280. Also, the parallel biometric signal processor 200 includes a port switching fabric 211 to connect the k ports 201 through 203 and the AFE unit 220, an AFE switching fabric 212 to connect the AFE unit 220 and the ADC unit 230, an ADC switching fabric 213 to connect the ADC unit 230 and the digital preprocessor 240, and a digital signal processor (DSP) switching fabric 214 to connect the digital preprocessor 240 and the feature extractor 250. The AFE unit 220 includes m amplifiers, for example, 221 through 224. The ADC unit 230 includes n converters, for example, 231 through 234. The digital preprocessor 240 includes p preprocessors, for example, 241 through 244. The feature extractor 250 includes q feature extractors, for example, 251 through 254.

The k ports 201 through 203 are connected to a sensor, for example, an electrode sensor to measure a potential difference of human body portions, a photochemical sensor, such as an ISFET, and a photoelectric sensor, for example, a photodiode sensor, to detect emission, absorption, fluorescence, and reflection of light. The k ports 201 through 203 are connected to other sensors in addition to the electrode sensor, the photochemical sensor, and the photoelectric sensor. The switching controller 260 selects at least one target port suitable for desired biometric information from among the k ports 201 through 203. The target port receives the biometric information from the sensor connected to the target port and transmits the received biometric information to at least one of the m amplifiers 221 through 224.

The m amplifiers 221 through 224 are disposed in parallel, and amplify a biometric signal input from at least one of the k ports 201 through 203 based on an amplifying attribute. The switching controller 260 selects at least one amplifier as a target amplifier from among the m amplifiers 221 through 224 in accord with desired biometric information. The target amplifier may amplify the biometric signal based on an amplifying attribute of the target amplifier. In an example, the m amplifiers 221 through 224 may have different amplifying attributes. In another example, the m amplifiers 221 through 224 may be a programmable amplifier that adjusts the amplifying attribute such as an input impedance, a bandwidth, and an amplification gain. Also, the m amplifiers 221 through 224 may include at least one of an IA, a PGA, and a BPF. A switching fabric is disposed among the m amplifiers 221 through 224. The switching controller 260 controls the switching fabric disposed among the m amplifiers 221 through 224.

In one illustrative configuration, the n converters 231 through 234 in the ADC unit 230 are arranged in parallel, and convert an amplifying signal from at least one of the m amplifiers 221 through 224 in the AFE unit 220 to a digital signal based on a converting attribute. The converting attribute includes at least one of an input dynamic range and an output bit resolution. The n converters 231 through 234 include different input dynamic ranges and different output bit resolutions. The switching controller 260 selects a converter suitable for desired biometric information as a target converter from among the n converters 231 through 234. The target converter converts the amplifying signal to the digital signal based on a converting attribute of the target converter.

The p preprocessors 241 through 244 in the digital preprocessor 240 are arranged in parallel, and preprocess a converted signal from at least one of the n converters 231 through 234 based on a preprocessing attribute. The preprocessing attribute may include at least one of an attribute of filtering an unnecessary frequency band of the converted signal and an attribute of extracting at least one set of preprocessing information on the converted signal. The preprocessing information may include at least one of information on time at which the converted signal is acquired and information on a frequency characteristic of the converted signal. For example, a preprocessing attribute of a first preprocessor 241 is the attribute of filtering the unnecessary frequency band of the converted signal. The first preprocessor 241 includes a high pass filter (HPF), a BPF, or a low pass filter (LPF). The HPF, the BPF, or the LPF may be implemented through a finite impulse response (FIR) and an infinite impulse response (IIR). In another example, a preprocessing attribute of a second preprocessor 242 is an attribute of extracting the information on time at which the converted signal is acquired. The second preprocessor 242 stores a local real time clock (RTC) value of a point in time at which a sample of the converted signal is acquired along with the converted signal. For still another example, a preprocessing attribute of a third preprocessor 243 may be the attribute of extracting the information on the frequency characteristic of the converted signal. The third preprocessor 243 may extract the information on the frequency characteristic of the converted signal using a fast Fourier transform (FFT). The switching controller 260 selects at least one preprocessor associated with desired biometric information as a target preprocessor from among the p preprocessors 241 through 244. The target preprocessor preprocesses the converted signal based on a preprocessing attribute of the target preprocessor.

The q feature extractors 251 through 254 are connected in parallel and at least one of the q feature extractors 251 through 254 extract at least one set of the biometric information from an output signal of at least one of the p preprocessors 241 through 244. For example, a first feature extractor 251 extracts information about a heart rate, a second feature extractor 252 extracts blood pressure information, a third feature extractor 253 extracts information of usage of a muscle, and a fourth feature extractor 254 extracts information about a brain activity. The switching controller 260 selects at least one feature extractor that may extract desired biometric information as a target feature extractor from among the q feature extractors 251 through 254. The target feature extractor extracts the biometric information from the output signal of the target preprocessor.

The switching controller 260 controls the port switching fabric 211, the AFE switching fabric 212, the ADC switching fabric 213, and the DSP switching fabric 214 to perform routing of a biometric signal received at the target port. The biometric signal includes desired biometric information to be extracted. The switching controller 260 controls the port switching fabric 211 so that the target amplifier in the AFE unit 220 receives the biometric signal from the target port. The switching controller 260 controls the AFE switching fabric 212 so that the target converter in the ADC unit 230 receives the amplifying signal from the target amplifier. The switching controller 260 controls the ADC switching fabric 213 so that the target preprocessor at the digital preprocessor 240 receives the converted signal from the target converter. The switching controller 260 controls the DSP switching fabric 214 so that the target feature extractor at the feature extractor unit 250 receives an output signal from the target preprocessor.

For example, an electrode sensor is attached to a human body to extract the information on a usage of a muscle, and a photodiode sensor and a pressure sensor are attached to the human body to extract heart rate information. In one illustrative example, the electrode sensor senses an EMG signal, the photodiode sensor senses a PPG signal, and the pressure sensor senses a pressure signal. The parallel biometric signal processor 200 simultaneously processes the EMG signal, the PPG signal, and the pressure signal to simultaneously provide the information on the usage of the muscle and the heart rate information. To process the EMG signal, the switching controller 260 performs the routing through a first port 201, a first amplifier 221, a second converter 232, a first preprocessor 241, and a third feature extractor 253. To process the PPG signal, the switching controller 260 routes the PPG signal through a second port 202, a second amplifier 222, a third converter 233, a second preprocessor 242, and a first feature extractor 251. Similarly, to process the pressure signal, the switching controller 260 routes the pressure signal through a third port 203, a third amplifier 223, a first converter 231, a third preprocessor 243, and the first feature extractor 251. The third feature extractor 253 extracts the information on the usage of the muscle from the preprocessed EMG signal, and the first feature extractor 251 extracts the heart rate information from the preprocessed PPG signal and the preprocessed pressure signal. As described in the foregoing, the parallel biometric signal processor 200 processes multiple biometric signals using a single chip. Accordingly, when the parallel biometric signal processor 200 is used as a sensor, a low-power small-sized sensor is implemented. Also, the parallel biometric signal processor 200 processes multiple biometric signals without an additional algorithm to process a biometric signal.

The register controller 270 controls detailed attributes of the m amplifiers 221 through 224, the n converters 231 through 234, the p preprocessors 241 through 244, and the q feature extractors 251 through 254.

The power controller 280 controls power to be provided to the m amplifiers 221 through 224, the n converters 231 through 234, the p preprocessors 241 through 244, and the q feature extractors 251 through 254. For example, when the switching controller 260 performs the routing through the first amplifier 221, the first converter 231, the first preprocessor 241, and the first feature extractor 251, the power controller 280 supplies the power to the first amplifier 221, the first converter 231, the first preprocessor 241, and the first feature extractor 251. The power controller 280 also blocks the supply of the power to remaining amplifiers, remaining converters, remaining preprocessors, and remaining feature extractors.

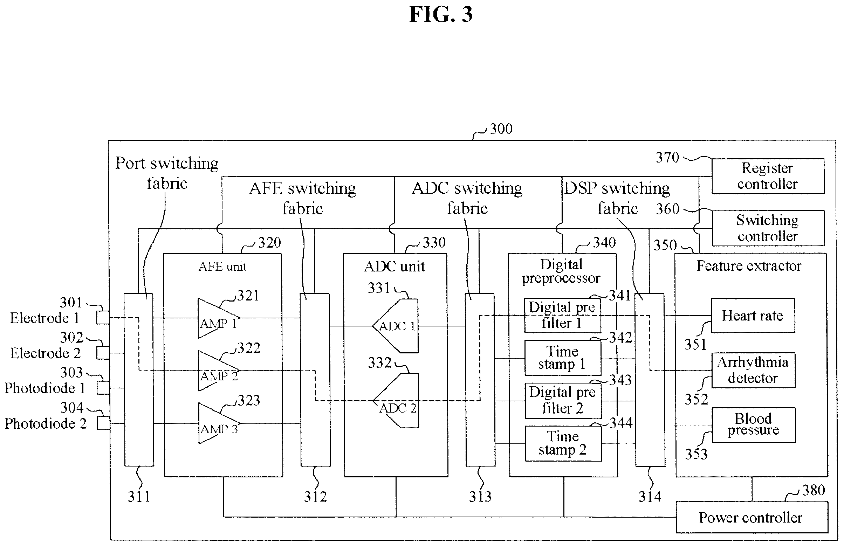

FIGS. 3 through 5 are diagrams illustrating examples of an operation of a parallel biometric signal processor, in accordance with various configurations.

FIG. 3 is a diagram illustrating an example of an operation of a parallel biometric signal processor 300 to extract arrhythmia detection information at a low power operating mode, in accordance with an embodiment.

Referring to FIG. 3, the parallel biometric signal processor 300 includes four ports, ports 301 through 304, an AFE unit 320, an ADC unit 330, a digital preprocessor 340, a feature extractor 350, a switching controller 360, a register controller 370, and a power controller 380. Also, the parallel biometric signal processor 300 includes a port switching fabric 311 to connect the four ports 301 through 304 and the AFE unit 320, an AFE switching fabric 312 to connect the AFE unit 320 and the ADC unit 330, an ADC switching fabric 313 to connect the ADC unit 330 and the digital preprocessor 340, and a DSP switching fabric 314 to connect the digital preprocessor 340 and the feature extractor 350. A first port 301 is connected to a first electrode sensor, a second port 302 is connected to a second electrode sensor, a third port 303 is connected to a first photodiode sensor, and a fourth port 304 is connected to a second photodiode sensor.

In one illustrative example, the AFE unit 320 includes three amplifiers, for example, 321 through 323. A first amplifier 321 is a high-performance amplifier having an amplification gain of 6 decibels (dB) and a common mode rejection ratio (CMRR) of -115 dB. A second amplifier 322 and a third amplifier 323 are a low-power amplifier having an amplification gain of 1 dB and a CMRR of -100 dB. The ADC unit 330 includes two converters, for example, 331 and 332. A first converter 331 may be a delta-sigma ADC unit having a 24 bit resolution, and a second converter 332 may be a successive approximation register (SAR) ADC unit having a 12 bit resolution. The digital preprocessor 340 includes four preprocessors, for example, 341 through 344. A first preprocessor 341 and a third preprocessor 343 filter an unnecessary frequency band of a converted signal, and a second preprocessor 342 and a fourth preprocessor 344 extract information on time at which the converted signal is acquired. The feature extractor 350 includes three feature extractors, for example, 351 through 353. A first feature extractor 351 extracts information on heart rates from a preprocessed biometric signal, a second feature extractor 352 extracts information on arrhythmia detection from a preprocessed biometric signal, and a third feature extractor 353 extracts blood pressure information from a preprocessed biometric signal.

To detect an arrhythmia, the switching controller 360 routes at least one signal through a target port, a target amplifier, a target converter, a target preprocessor, and a target feature extractor. The switching controller 360 selects, as the target port, the first port 301 connected to the first electrode sensor sensing a desirable quality ECG signal from between the first electrode sensor and the second electrode sensor. The switching controller 360 selects the second amplifier 322 and the second converter 332 that consumes a lower amount of power than the target amplifier and the target converter, respectively. Also, the switching controller 360 selects the first preprocessor 341 as the target preprocessor to eliminate a frequency band exceeding 40 Hz, and selects the second feature extractor 352 that extracts the information on the detection of the arrhythmia as the target feature extractor. As indicated by a dotted line, the switching controller 360 performs the routing of a signal through the first port 301, the second amplifier 322, the second converter 332, the first preprocessor 341, and the second feature extractor 352 by controlling the port switching fabric 311, the AFE switching fabric 312, the ADC switching fabric 313, and the DSP switching fabric 314.

Accordingly, the first port 301 receives the ECG signal from the first electrode sensor and transmits the ECG signal to the second amplifier 322. The second amplifier 322 amplifies the received ECG signal based on the amplification gain of 1 dB and the CMRR of -100 dB. The second converter 332 having the 12 bit resolution, for instance, samples an amplifying signal using a sampling frequency of 256 Hz. The ECG signal may include significant information in a frequency band of 0 through 40 Hz. The first preprocessor 341 changes a number of taps and a coefficient of a digital filter and filter a frequency band exceeding 40 Hz of the converted ECG signal. The second feature extractor 352 detects the arrhythmia from the filtered ECG signal in real time.

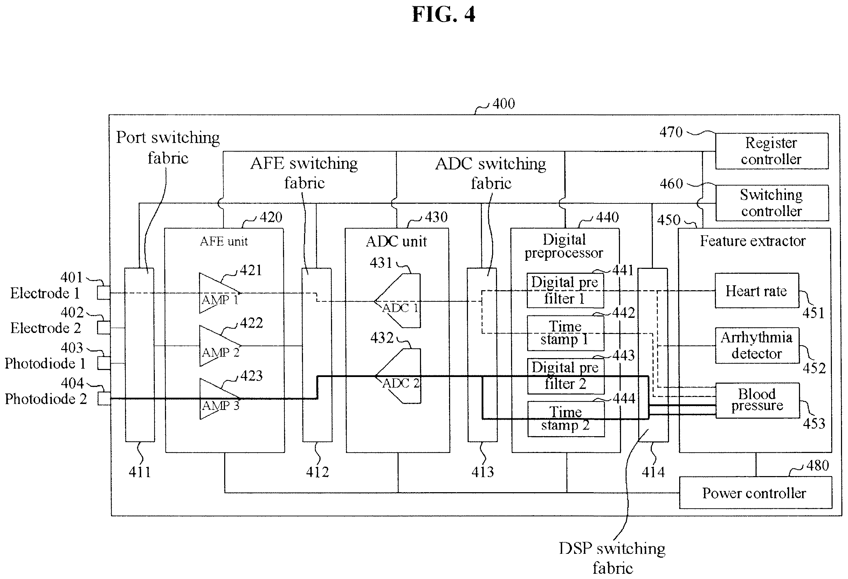

FIG. 4 is a diagram illustrating an example of an operation of a parallel biometric signal processor 400 to extract arrhythmia detection information in a high precision operating mode.

Referring to FIG. 4, the parallel biometric signal processor 400 includes four ports, for example, 401 through 404, an AFE unit 420, an ADC unit 430, a digital preprocessor 440, a feature extractor 450, a switching controller 460, a register controller 470, a power controller 480, and four switching fabrics, for example, 411 through 414. Characteristics of a first amplifier 421 through a third amplifier 423, a first converter 431 and a second converter 432, a first preprocessor 441 through a fourth preprocessor 444, and a first feature extractor 451 through a third feature extractor 453 may be the same as characteristics of the first amplifier 321 through the third amplifier 323, the first converter 331 and the second converter 332, the first preprocessor 341 through the fourth preprocessor 344, and the first feature extractor 351 through the third feature extractor 353 illustrated in FIG. 3.

When blood pressure and heart rate information is provided along with the arrhythmia detection information, medical diagnostic accuracy may increase. The heart rate information and the arrhythmia detection information may be extracted from an ECG signal, and the blood pressure information be extracted from ECG information and a PPG signal. Accordingly, the parallel biometric signal processor 400 processes the ECG signal using a first routing path and the PPG signal using a second routing path. In an example, the parallel biometric signal processor 400 simultaneously processes the first routing path and the second routing path to simultaneously extract the arrhythmia detection information, the blood pressure information, and the heart rate information.

To process the ECG signal based on the first routing path, the switching controller 460 performs routing on a target port, a target amplifier, a target converter, a first target preprocessor, a second target preprocessor, and a first target feature extractor through a third target feature extractor by controlling a port switching fabric 411, an AFE switching fabric 412, an ADC switching fabric 413, and a DSP switching fabric 414. The switching controller 460 selects, as the target port, a first port 401 connected to a first electrode sensor sensing a desirable quality ECG signal from between the first electrode sensor and a second electrode sensor. The switching controller 460 selects, as the target amplifier, a first amplifier 421 having a high amplification factor and a desirable power to control noise. The switching controller 460 may select, as the target converter, a first converter 431 that accurately converts an amplifying signal. Also, the switching controller 460 selects a first preprocessor 441 as the first target preprocessor to eliminate a frequency band exceeding 40 Hz and selects a second preprocessor 442 as the second target preprocessor to extract information on a time at which a converted signal is acquired. The switching controller 460 selects, as the first target feature extractor, a first feature extractor 451 to extract the information on the heart rates. The switching controller 460 also selects, as the second target feature extractor, a second feature extractor 452 to extract the arrhythmia detection information, and selects, as the third target feature extractor, a third feature extractor 453 to extract the blood pressure information. Along the first routing path as indicated by a dotted line, the first port 401 receives the ECG signal from the first electrode sensor and transmits the ECG signal to the first amplifier 421. The first amplifier 421 amplifies the received ECG signal based on an amplification gain of 6 dB and a CMRR of -115 dB. The first converter 431 having a 24 bit resolution samples the amplified ECG signal with a sampling frequency of 1024 Hz or 2048 Hz. The ECG signal may include significant information in a frequency band in a range of 0 through 40 Hz. Thus, the first preprocessor 441 changes a number of taps and a coefficient of a digital filter and filter a frequency band exceeding 40 Hz of the sampled ECG signal. The second preprocessor 442 extracts a local RTC value at a point in time at which a sample of the sampled ECG signal from the first converter 431 is acquired. The first feature extractor 451 extracts the heart rate information from the preprocessed ECG signal, and the second feature extractor 452 detects an arrhythmia from the preprocessed ECG signal. The third feature extractor 453 extracts the blood pressure information from the preprocessed ECG signal, a local RTC value of the ECG signal, a preprocessed PPG signal obtained through the second routing path, and a local RTC value of the PPG signal.

To process the PPG signal based on the second routing path, the switching controller 460 performs the routing of a signal through the target port, the target amplifier, the target converter, the first target preprocessor, the second target preprocessor, and the target feature extractor by controlling the port switching fabric 411, the AFE switching fabric 412, the ADC switching fabric 413, and the DSP switching fabric 414. The switching controller 460 selects, as the target port, the fourth port 404 connected to a second photodiode sensor sensing a desirable quality PPG signal from between a first photodiode sensor and the second photodiode sensor. The switching controller 460 selects the third amplifier 423 and the second converter 432 that consumes a lower amount of power than the target amplifier and the target converter. Also, the switching controller 460 selects the third preprocessor 443 as the first target preprocessor to eliminate a frequency band exceeding 5 Hz, and the fourth preprocessor 444 as the second target preprocessor to extract the information at a point in time at which the converted signal is acquired. The switching controller 460 selects, as the target feature extractor, the third feature extractor 453 to extract the blood pressure information.

Along the second routing path as indicated by a bold line in FIG. 4, the fourth port 404 may receive the PPG signal from the second photodiode sensor and transmit the PPG signal to the third amplifier 423. The third amplifier 423 may amplify the received PPG signal based on an amplification gain of 1 dB and a CMRR of -100 dB. The second converter 432 having a 12 bit resolution may sample an amplifying signal with a sampling frequency of 256 Hz. The third preprocessor 443 may change a number of taps and a coefficient of a digital filter and filter the frequency band exceeding 40 Hz of the sampled PPG signal. The fourth preprocessor 444 may extract a local RTC value of a point in time at which a sample of a converted signal of the second converter 432 is acquired. The third feature extractor 453 extracts the blood pressure information from the preprocessed ECG signal obtained through the first routing path, the local RTC value of the ECG signal, the preprocessed PPG signal, and the local RTC value of the PPG signal.

In accordance with an embodiment, the parallel biometric signal processor 400 simultaneously processes the first routing path and the second routing path, and externally transmits the information on the heart rates, the arrhythmia detection information, and the blood pressure information extracted through the first routing path and the second routing path.

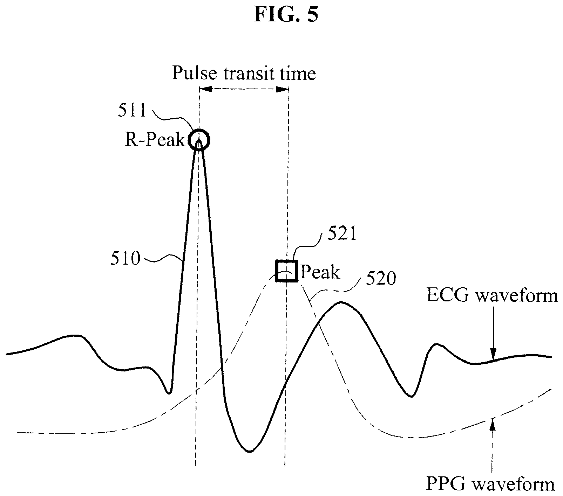

FIG. 5 is a diagram illustrating an example of an operation of the third feature extractor 453 of FIG. 4.

Referring to FIG. 5, the third feature extractor 453 obtains an ECG signal 510 and a local RTC value of the ECG signal 510 through the first routing path, and obtains a PPG signal 520 and a local RTC value of the PPG signal 520 through the second routing path. The third feature extractor 453 extracts an R peak value 511 having a highest numerical value from the ECG signal 510, and extracts a peak value 521 having a highest numerical value from the PPG signal 520. The third feature extractor 453 calculates a pulse transit time (PTT) indicating a time difference between the R peak value 511 and the peak value 521. The third feature extractor 453 extracts blood pressure information without use of a cuff system based on the calculated PTT.

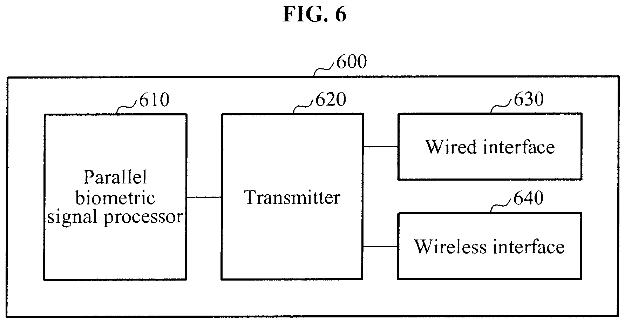

FIG. 6 is a block diagram illustrating an example of an integrated biomedical signal processor 600.

Referring to FIG. 6, the integrated biomedical signal processor 600 includes a parallel biometric signal processor 610, a transmitter 620, a wired interface 630, and a wireless interface 640.

The parallel biometric signal processor 610 includes amplifiers to amplify at least one biometric signal into at least one amplifying attribute, and converters to convert an amplifying signal of the amplifiers to at least one converting attribute. The parallel biometric signal processor 610 further includes preprocessors to preprocess a converted signal of the converters based on at least one preprocessing attribute, feature extractors to extract at least one set of biometric information from an output signal form the preprocessors, and switching fabrics to be connected among the amplifiers, the converters, the preprocessors, and the feature extractors.

Also, the parallel biometric signal processor 610 may include a switching controller (as shown and discussed with respect to FIGS. 2 to 4) to control the switching fabrics and perform routing on at least one target amplifier to which the at least one biometric signal is input, at least one target converter to which an amplifying signal of the target amplifier is input, at least one preprocessor to which a converted signal of the target converter is input, and at least one feature extractor to which an output signal of the target preprocessor is input. Through the routing by the switching controller, the parallel biometric signal processor 610 extracts the biometric information from a biometric signal.

The transmitter 620 externally transmits the biometric information extracted by the parallel biometric signal processor 610 through the wired interface 630 or the wireless interface 640. For example, the biometric information extracted by the parallel biometric signal processor 610 is packetized and externally transmitted. The wired interface 630 includes at least one of an UART, an SPI, and an I2C. The wireless interface 640 may include at least one of BAN, Bluetooth, ZigBee, and NFC.

Descriptions of the parallel biometric signal processors provided with reference to FIGS. 1 through 5 may be identically applied to the parallel biometric signal processor 610 illustrated in FIG. 6 and; thus, a detailed and repeated description will be omitted here for brevity.

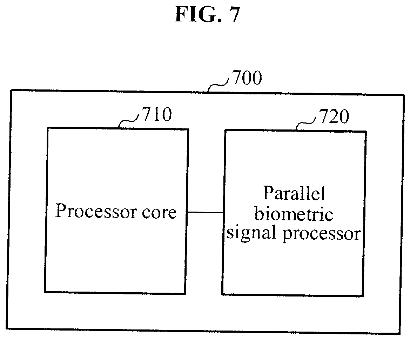

FIG. 7 is a block diagram illustrating an example of an application processor 700.

Referring to FIG. 7, the application processor 700 includes a processor core 710 and a parallel biometric signal processor 720.

The processor core 710 processes commands and data.

The parallel biometric signal processor 720 includes amplifiers to amplify at least one biometric signal into at least one amplifying attribute, converters to convert an amplifying signal of the amplifiers to at least one converting attribute, preprocessors to preprocess a converted signal of the converters based on at least one preprocessing attribute, feature extractors to extract at least one set of biometric information from an output signal of the preprocessors, and switching fabrics to be connected among the amplifiers, the converters, the preprocessors, and the feature extractors. Also, the parallel biometric signal processor 720 includes a switching controller to control the switching fabrics to perform routing on at least target amplifier to which at least one biometric signal is input. The parallel biometric signal processor 720 also includes at least one target converter to which an amplifying signal of the target amplifier is input, at least one target preprocessor to which a converted signal of the target converter is input, and at least one target feature extractor to which an output signal of the target preprocessor is input. The switching controller performs the routing on the target amplifier, the target converter, the target preprocessor, and the target feature extractor in accordance with a control by the processor core 710.

Descriptions of the parallel biometric signal processors provided with reference to FIGS. 1 through 5 may be identically applied to the parallel biometric signal processor 720 illustrated in FIG. 7 and thus, a detailed and repeated description will be omitted here for brevity.

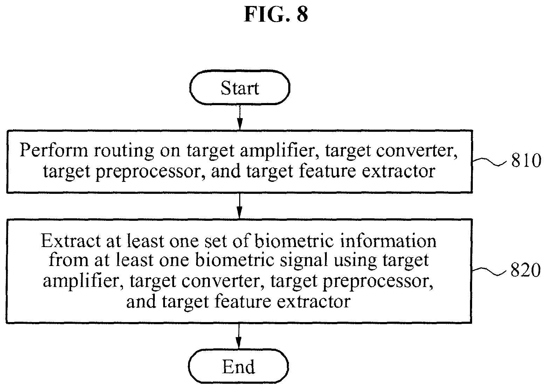

FIG. 8 is a flowchart illustrating an example of a method of controlling a parallel biometric signal processor.

Referring to FIG. 8, at operation 810, a controller of the parallel biometric signal processor performs routing, from amplifiers, converters, preprocessors, and feature extractors included in the parallel biometric signal processor, on at least one target amplifier to which at least one biometric signal is input, at least one target converter to which an amplifying signal of the target amplifier is input, at least one target preprocessor to which a converted signal of the target converter is input, and at least one target feature extractor to which an output signal of the target preprocessor is input.

At operation 820, the controller of the parallel biometric signal processor extracts at least one set of biometric information from the at least one biometric signal using the target amplifier, the target converter, the target preprocessor, the target feature extractor on which the routing is performed.

Descriptions of the functions performed by the structural elements of the parallel biometric signal processor as illustrated and described with regards to FIGS. 1 through 7 may be identically applied to the method of controlling the parallel biometric signal processor described with reference to FIG. 8 and; thus, a detailed and repeated description will be omitted here for brevity.

In accordance with an illustrative example, a computer program embodied on a non-transitory computer-readable medium may also be provided, encoding instructions to perform at least the method described in FIG. 8.

Program instructions to perform a method described in FIG. 8, or one or more operations thereof, may be recorded, stored, or fixed in one or more computer-readable storage media. The program instructions may be implemented by a computer. For example, the computer may cause a processor to execute the program instructions. The media may include, alone or in combination with the program instructions, data files, data structures, and the like. Examples of computer-readable media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The program instructions, that is, software, may be distributed over network coupled computer systems so that the software is stored and executed in a distributed fashion. For example, the software and data may be stored by one or more computer readable recording mediums. Also, functional programs, codes, and code segments for accomplishing the example embodiments disclosed herein may be easily construed by programmers skilled in the art to which the embodiments pertain based on and using the flow diagrams and block diagrams of the figures and their corresponding descriptions as provided herein.

The units described herein may be implemented using hardware components. For example, the hardware components may include controllers, microphones, amplifiers, bandpass filters, audio to digital convertors, and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A parallel biometric signal processor (200), comprising:
ports (201-203) connected to at least one sensor configured to sense at least one biometric signal;
amplifiers (221-224) configured to amplify the at least one biometric signal based on an amplifying attribute;
converters (231-234) configured to convert the amplified signal to a converted signal based on a converting attribute;
preprocessors (241-244) configured to preprocess the converted signal based on a preprocessing attribute; and
feature extractors (251-254) configured to extract a set of biometric information from an output signal of the preprocessors;
**the parallel biometric signal processor (200) further comprising**
a switching controller (260) configured to select a target port from among the ports (201-203), a target amplifier from among the amplifiers (221-224), a target converter from among the converters (231-234), a target preprocessor from among the preprocessors (241-244) and a target feature extractor from among the feature extractors (251-254) to extract a target biometric information; and
switching fabrics (211-214) connected among the ports (201-203), the amplifiers (221-224), the converters (231-234), the preprocessors (241-244), and the feature extractors (251-254),
wherein a first switching fabric (211) connects the ports (210-203) and the amplifiers (221-224), a second switching fabric (212) connects the amplifiers (221-224) and the converters (231-234), a third switching fabric (213) connects the converters (231-234) and the preprocessors (241-244) and a fourth switching fabric (214) connects the preprocessors (241-244) and the feature extractors (251-254),
such that the switching controller (260) is configured to control the first to fourth switching fabrics (211- 214) to perform routing through the target port, the target amplifier, the target converter, the target preprocessor and the target feature extractor of the at least one biometric signal received at the target port.

2. The processor of claim 1, wherein the switching controller (260) is configured to perform rerouting through the target amplifier, the target converter, the target preprocessor, and the target feature extractor; or
wherein the switching controller (260) is configured to perform the routing through the target amplifier, the target converter, the target preprocessor, and the target feature extractor based on an operating mode.

3. The processor of claim 2, wherein the operating mode comprises one of a low power operating mode and a high precision operating mode.

4. The processor of claim 1, wherein the amplifiers (221-224), the converters (231-234), the preprocessors (241-244), and the feature extractors (251-254) are connected in parallel, or
wherein the amplifiers (221-224) possess different amplified attributes,
wherein the converters (231-234) comprise different converting attributes,
wherein the preprocessors (241-244) comprise different preprocessing attributes, and
wherein the feature extractors (251-254) are configured to extract different sets of the biometric information.

5. The processor of claim 2,
wherein the sensor comprises at least one of an electrode sensor, a photochemical sensor, and a photoelectric sensor.

6. The processor of claim 1, wherein the amplified attribute comprises at least one of an input impedance, a bandwidth, and an amplification gain, or
wherein the amplifiers (221-224) are configured to adjust the amplified attribute, or
wherein the amplifiers (221-224) comprise at least one of an instrument amplifier, IA, a programmable gain amplifier, PGA, and a band pass filter, BPF, or
wherein the converters (231-234) are configured to convert the amplified signal to a digital signal based on the converting attribute, or
wherein the converting attribute comprises at least one of an input dynamic range and an output bit resolution, or
wherein the preprocessing attribute comprises at least one of an attribute of filtering an unnecessary frequency band of the converted signal and an attribute of extracting a set of preprocessing information from the converted signal, said at least one set of preprocessing information comprising at least one of information on a time at which the converted signal is acquired and information on a frequency characteristic of the converted signal.

7. The processor of claim 1, further comprising:
a power controller (280) configured to control power to be provided to the amplifiers (221-224), the converters (231-234), the preprocessors (241-244), and the feature extractors (251-254), or
a register controller (270) configured to control detailed attributes of the amplifiers (221-224), the converters (231-234), the preprocessors (231-234), and the feature extractors (251-254), or
a transmitter (620) configured to transmit the biometric information to an external device.

8. The processor of claim 7, further comprising:
an interface wiredly (630) or wirelessly (640) connected to the external device,
wherein the transmitter (620) is configured to transmit the biometric information to the external device using the interface (630, 640) and/or
wherein the interface wiredly (630) connected to the external device comprises at least one of a universal asynchronous receiver transmitter, UART, a serial peripheral interface, SPI, and an inter-integrated circuit, I2C, and
wherein the interface wirelessly (640) connected to the external device comprises at least one of body area network, BAN, Bluetooth, ZigBee, and near field communication, NFC.

9. An application processor (700), comprising:
a processor core (710) configured to process commands and data; and
a parallel biometric signal processor (720) of claim 1 configured to extract a set of biometric information from a biometric signal.

10. A computer-readable medium having instructions that, when performed by the processor of claim 1,
cause the processor to perform the steps of:
performing routing through ports (201-203), amplifiers (221-224), converters (231-234), preprocessors (241-244), and feature extractors (251-254),
receive a biometric signal through a target port of the target ports (201-203);
amplifying the biometric signal through a target amplifier of the amplifiers (221-224); converting the amplified biometric signal through a target converter of the converters (231-234);
preprocessing the converted signal through a target preprocessor of the preprocessors (241-244); and
extracting a set of biometric information from the preprocessed signal through a feature extractor of the feature extractors (251-254);
the performing comprises selecting the target port from among the ports (201-203), the target amplifier from among the amplifiers (221-224), the target converter from among the converters (231-234), the target preprocessor from among the preprocessors (241-244) and the target feature extractor from among the feature extractors (251-254) to extract a target biometric information; and further connecting the ports (201-203), the amplifiers (221-224), the converters (231-234),
the preprocessors (241-244), and the feature extractors (251-254) through switching fabrics (211-214),
wherein a first switching fabric (211) connects the ports (210-203) and the amplifiers (221-224), a second switching fabric (212) connects the amplifiers (221-224) and the converters (231-234), a third switching fabric (213) connects the converters (231-234) and the preprocessors (241-244) and a fourth switching fabric (214) connects the preprocessors (241-244) and the feature extractors (251-254),
for controlling the first to fourth switching fabrics (211- 214) to perform routing through the target port, the target amplifier, the target converter, the target preprocessor and the target feature extractor of the biometric signal received at the target port.

11. A method of operating a parallel biometric signal processor, comprising the steps of:
receiving, by a controller, a first and a second signal, and simultaneously processing the first and the second signals by simultaneously routing the first signal through a first path and the second signal through a second path,
converting, through the first path, by the controller, the first signal to a first converted signal using a first converting attribute, preprocessing the first converted signal based on a first preprocessing attribute, and extracting first biometric information, and
converting, through the second path, by the controller, the second signal to a second converted signal using a second converting attribute, preprocessing the second converted signal based on a second preprocessing attribute, and extracting second biometric information; wherein
the receiving comprises selecting a target amplifier, from among amplifiers (221-224), corresponding to each of the first path and the second path, a target converter, from among converters (231-234), corresponding to each of the first path and the second path, a target preprocessor, from among preprocessors (241-244) corresponding to each of the first path and the second path, and a target feature extractor, from among feature extractors (251-254), corresponding to each of the first path and the second path, and
connecting the amplifiers (221-224), the converters (231-234), the preprocessors (241-244), and the feature extractors (251-254) through switching fabrics (212-214) corresponding to each of the first path and the second path,
wherein a first switching fabric (212) connects the amplifiers (221-224) and the converters (231-234), a second switching fabric (213) connects the converters (231-234) and the preprocessors (241-244) and a third switching fabric (214) connects the preprocessors (241-244) and the feature extractors (251-254) corresponding to each of the first and second path,
thereby controlling the first to third switching fabrics (212-214) to perform routing through the target amplifier, the target converter, the target preprocessor and the target feature extractor of the first and second signal corresponding to each of the first and second path.

12. The method of claim 11, wherein the controller is further configured to amplify the first
signal using a first amplifying attribute and the second signal using a second amplifying attribute prior to converting the first and the second signals, or
further comprising amplifying, by the controller, the first and the second signals, converting, by converters, the amplified first signal to the first converted signal and the amplified second signal to the second converted signal, preprosessing, by preprocessors, the first converted signal and the second converted signal, and extracting, by feature extractors, the first and the second biometric information.

13. The method of claim 11, wherein the first and the second amplifying attributes comprise at least one of an input impedance, a bandwidth, and an amplification gain, the first and the second converting attributes comprise at least one of an input dynamic range and an output bit resolution, and the first and the second preprocessing attributes comprise at least one of an attribute of filtering an unnecessary frequency band and an attribute of extracting at least one set of preprocessing information.

## Patentansprüche

1. Ein paralleler biometrischer Signalprozessor (200) umfassend:
Anschlüsse (201-203), die mit mindestens einem Sensor verbunden sind, der so konfiguriert ist, dass er mindestens ein biometrisches Signal erfasst;
Verstärker (221-224), die so konfiguriert sind, dass sie das mindestens eine biometrische Signal auf Grundlage eines Verstärkungsmerkmals verstärken;
Wandler (231-234), die so konfiguriert sind, dass sie das verstärkte Signal auf Grundlage eines Umwandlungsmerkmals in ein umgewandeltes Signal umwandeln;
Vorprozessoren (241-244), die so konfiguriert sind, dass sie das umgewandelte Signal auf Grundlage eines Vorverarbeitungsmerkmals vorverarbeiten; und
Merkmalsextraktoren (251-254), die konfiguriert sind, dass sie einen Satz biometrischer Informationen aus einem Ausgangssignal der Vorprozessoren extrahieren;
**der parallele biometrische Signalprozessor (200) ferner umfassend**
eine Schaltsteuerung (260), die so konfiguriert ist, dass sie einen Zielanschluss unter den Anschlüssen (201-203), einen Zielverstärker unter den Verstärkern (221-224), einen Zielwandler unter den Wandlern (231-234), einen Zielvorprozessor unter den Vorprozessoren (241-244) und einen Zielmerkmalsextraktor unter den Merkmalsextraktoren (251-254) auswählt, um eine biometrische Zielinformation zu extrahieren; und
Schalteinrichtungen (211-214), die mit den Anschlüssen (201-203), den Verstärkern (221-224), den Wandlern (231-234), den Vorprozessoren (241-244) und den Merkmalsextraktoren (251-254) verbunden sind,
wobei eine erste Schalteinrichtung (211) die Ports (210-203) und die Verstärker (221-224) verbindet, eine zweite Schalteinrichtung (212) die Verstärker (221-224) und die Wandler (231-234) verbindet, eine dritte Schalteinrichtung (213) die Wandler (231-234) und die Vorprozessoren (241-244) verbindet und eine vierte Schalteinrichtung (214) die Vorprozessoren (241-244) und die Merkmalsextraktoren (251-254) verbindet,
so dass die Schaltsteuerung (260) so konfiguriert ist, dass sie die erste bis vierte Schalteinrichtung (211-214) steuert, um ein Routing durch den Zielanschluss, den Zielverstärker, den Zielwandler, den Zielvorprozessor und den Zielmerkmalsextraktor des mindestens einen biometrischen Signals, das am Zielanschluss empfangen wird, durchzuführen.

2. Der Prozessor nach Anspruch 1, wobei die Schaltsteuerung (260) so konfiguriert ist, dass sie ein Re-Routing durch den Zielverstärker, den Zielwandler, den Zielvorprozessor und den Zielmerkmalsextraktor durchführt; oder
wobei die Schaltsteuerung (260) so konfiguriert ist, dass sie das Routing durch den Zielverstärker, den Zielwandler, den Zielvorprozessor und den Zielmerkmalsextraktor auf Grundlage eines Betriebsmodus durchführt.

3. Der Prozessor nach Anspruch 2, wobei der Betriebsmodus entweder einen Betriebsmodus mit geringer Leistung oder einen Betriebsmodus mit hoher Präzision umfasst.

4. Der Prozessor nach Anspruch 1, wobei die Verstärker (221-224), die Wandler (231-234), die Vorprozessoren (241-244) und die Merkmalsextraktoren (251-254) parallel geschaltet sind, oder
wobei die Verstärker (221-224) unterschiedliche Verstärkereigenschaften besitzen, wobei die Wandler (231-234) unterschiedliche Wandlungsmerkmale aufweisen,
wobei die Vorprozessoren (241-244) unterschiedliche Vorverarbeitungsmerkmale aufweisen, und
wobei die Merkmalsextraktoren (251-254) so konfiguriert sind, dass sie verschiedene Sätze der biometrischen Informationen extrahieren.

5. Der Prozessor nach Anspruch 2,
wobei der Sensor mindestens einen von einem Elektrodensensor, einem photochemischen Sensor und einem photoelektrischen Sensor umfasst.

6. Der Prozessor nach Anspruch 1, wobei das Verstärkermerkmal mindestens eines von einer Eingangsimpedanz, einer Bandbreite und einem Verstärkungsfaktor umfasst, oder
wobei die Verstärker (221-224) zum Einstellen des Verstärkermerkmals konfiguriert sind, oder
wobei die Verstärker (221-224) mindestens eines von einem Instrumentenverstärker, IA, ein programmierbarer Leistungsverstärker, PGA, und einem Bandpassfilter, BPF, umfassen, oder
wobei die Wandler (231-234) so konfiguriert sind, dass sie das verstärkte Signal auf Grundlage des Umwandlungsmerkmals in ein digitales Signal umwandeln, oder
wobei das Umwandlungsmerkmal mindestens eines von einem Eingangsdynamikbereich und einer Ausgangsbitauflösung umfasst, oder
wobei das Vorverarbeitungsmerkmal mindestens eines von einem Merkmal zum Filtern eines unnötigen Frequenzbandes des umgewandelten Signals und einem Merkmal zum Extrahieren eines Satzes von Vorverarbeitungsinformationen aus dem umgewandelten Signal umfasst, der besagte mindestens eine Satz von Vorverarbeitungsinformationen mindestens eines von Informationen über eine Zeit, zu der das umgewandelte Signal erfasst wird, und Informationen über eine Frequenzeigenschaft des umgewandelten Signals umfasst.

7. Der Prozessor nach Anspruch 1, ferner umfassend:
einen Leistungsregler (280), der so konfiguriert ist, dass er die den Verstärkern (221-224), den Wandlern (231-234), den Vorprozessoren (241-244) und den Merkmalsextraktoren (251-254) bereitzustellende Leistung regelt, oder
einen Registerregler (270), der so konfiguriert ist, dass er detaillierte Merkmale der Verstärker (221-224), der Wandler (231-234), der Vorprozessoren (231-234) und der Merkmalsextraktoren (251-254) regelt, oder
einen Sender (620), der so konfiguriert ist, dass er die biometrischen Informationen an ein externes Gerät überträgt.

8. Der Prozessor nach Anspruch 7 ferner umfassend:
eine drahtgebundene (630) oder drahtlose (640) Schnittstelle, die mit dem externen Gerät verbunden ist,
wobei der Sender (620) so konfiguriert ist, dass er die biometrischen Informationen unter Verwendung der Schnittstelle (630, 640) an das externe Gerät überträgt und/oder
wobei die drahtgebundene Schnittstelle (630), die mit dem externen Gerät verbunden ist, mindestens einen universellen asynchronen Empfängertransmitter, UART, eine serielle periphere Schnittstelle, SPI, oder eine interintegrierte Schaltung, I2C, umfasst, und
wobei die Schnittstelle, die drahtlos (640) mit dem externen Gerät verbunden ist, mindestens eines aus Body Area Network, BAN, Bluetooth, ZigBee und Near Field Communication, NFC, umfasst.

9. Ein Anwendungsprozessor (700), umfassend:
einen Prozessorkern (710), der zur Verarbeitung von Befehlen und Daten konfiguriert ist; und
einen parallelen biometrischen Signalprozessor (720) nach Anspruch 1, der so konfiguriert ist, dass er einen Satz biometrischer Informationen aus einem biometrischen Signal extrahiert.

10. Ein computerlesbares Medium mit Anweisungen, die, wenn sie von dem Prozessor nach Anspruch 1 ausgeführt werden, den Prozessor veranlassen, die folgenden Schritte auszuführen:
Durchführen eines Routings durch Anschlüsse (201-203), Verstärker (221-224), Wandler (231-234), Vorprozessoren (241-244) und Merkmalsextraktoren (251-254),
Empfangen eines biometrischen Signals über einen Zielanschluss der Zielanschlüsse (201-203);
Verstärken des biometrischen Signals durch einen Zielverstärker der Verstärker (221-224);
Umwandeln des verstärkten biometrischen Signals durch einen Zielwandler der Wandler (231-234);
Vorverarbeiten des umgewandelten Signals durch einen Zielvorprozessor der Vorprozessoren (241-244); und
Extrahieren eines Satzes von biometrischen Informationen aus dem vorverarbeiteten Signal durch einen Merkmalsextraktor der Merkmalsextraktoren (251-254);
das Durchführen umfassend das Auswählen des Zielanschlusses aus den Anschlüssen (201-203), des Zielverstärkers aus den Verstärkern (221-224), des Zielwandlers aus den Wandlern (231-234), des Zielvorprozessors aus den Vorprozessoren (241-244) und des Zielmerkmalsextraktors aus den Merkmalsextraktoren (251-254), um eine biometrische Zielinformation zu extrahieren; und ferner
Verbinden der Anschlüsse (201-203), der Verstärker (221-224), der Wandler (231-234), der Vorprozessoren (241-244) und der Merkmalsextraktoren (251-254) durch Schalteinrichtungen (211-214),
wobei eine erste Schalteinrichtung (211) die Anschlüsse (210-203) und die Verstärker (221-224) verbindet, eine zweite Schalteinrichtung (212) die Verstärker (221-224) und die Wandler (231-234) verbindet, eine dritte Schalteinrichtung (213) die Wandler (231-234) und die Vorprozessoren (241-244) verbindet und eine vierte Schalteinrichtung (214) die Vorprozessoren (241-244) und die Merkmalsextraktoren (251-254) verbindet,
zum Steuern der ersten bis vierten Schalteinrichtung (211-214), um ein Routing durch den Zielanschluss, den Zielverstärker, den Zielwandler, den Zielvorprozessor und den Zielmerkmalsextraktor des empfangenen biometrischen Signals am Zielanschlussdurchzuführen.

11. Ein Verfahren zum Betreiben eines parallelen biometrischen Signalprozessors, umfassend die Schritte:
Empfangen eines ersten und eines zweiten Signals durch eine Steuerung und gleichzeitiges Verarbeiten des ersten und des zweiten Signals durch gleichzeitiges Routing des ersten Signals durch einen ersten Pfad und des zweiten Signals durch einen zweiten Pfad,
Umwandeln des ersten Signals über den ersten Pfad durch die Steuerung in ein erstes umgewandeltes Signal unter Verwendung eines ersten Umwandlungsmerkmals, Vorverarbeiten des ersten umgewandelten Signals basierend auf einem ersten Vorverarbeitungsmerkmal und Extrahieren erster biometrischer Informationen, und
Umwandeln des zweiten Signals über den zweiten Pfad durch die Steuerung in ein zweites umgewandeltes Signal unter Verwendung eines zweiten Umwandlungsmerkmals, Vorverarbeiten des zweiten umgewandelten Signals basierend auf einem zweiten Vorverarbeitungsmerkmals und Extrahieren zweiter biometrischer Informationen;
wobei
das Empfangen das Auswählen eines Zielverstärkers aus den Verstärkern (221-224), die jeweils dem ersten Pfad und dem zweiten Pfad entsprechen, eines Zielwandlers aus den Wandlern (231-234), die jeweils dem ersten Pfad und dem zweiten Pfad entsprechen, eines Zielvorprozessors aus den Vorprozessoren (241-244), die jeweils dem ersten Pfad und dem zweiten Pfad entsprechen, und eines Zielmerkmalsextraktors aus den Merkmalsextraktoren (251-254), die jeweils dem ersten Pfad und dem zweiten Pfad entsprechen, umfasst, und
Verbinden der Verstärker (221-224), der Wandler (231-234), der Vorprozessoren (241-244) und der Merkmalsextraktoren (251-254) durch Schalteinrichtungen (212-214), die jeweils dem ersten Pfad und dem zweiten Pfad entsprechen,
wobei eine erste Schalteinrichtung (212) die Verstärker (221-224) und die Wandler (231-234) verbindet, eine zweite Schalteinrichtung (213) die Wandler (231-234) und die Vorprozessoren (241-244) verbindet und eine dritte Schalteinrichtung (214) die Vorprozessoren (241-244) und die Merkmalsextraktoren (251-254) verbindet, die jeweils dem ersten und zweiten Pfad entsprechen,
wodurch die erste bis dritte Schalteinrichtung (212-214) gesteuert wird, um ein Routing durch den Zielverstärker, den Zielwandler, den Zielvorprozessor und den Zielmerkmalsextraktor des ersten und zweiten Signals, die jeweils dem ersten und zweiten Pfad entsprechen, durchzuführen.

12. Das Verfahren nach Anspruch 11, wobei die Steuerung ferner so konfiguriert ist, dass sie das erste Signal unter Verwendung eines ersten Verstärkungsmerkmals und das zweite Signal unter Verwendung eines zweiten Verstärkungsmerkmals verstärkt, bevor sie das erste und das zweite Signal umwandelt, oder
ferner umfassend das Verstärken des ersten und des zweiten Signals durch die Steuerung, das Umwandeln des verstärkten ersten Signals in das erste umgewandelte Signal und des verstärkten zweiten Signals in das zweite umgewandelte Signal durch Wandler, das Vorverarbeiten des ersten umgewandelten Signals und des zweiten umgewandelten Signals durch Vorprozessor und das Extrahieren der ersten und der zweiten biometrischen Information durch Merkmalsextraktoren.

13. Das Verfahren nach Anspruch 11, wobei das erste und das zweite Verstärkungsmerkmal mindestens eines von einer Eingangsimpedanz, einer Bandbreite und einem Verstärkungsfaktor umfassen, das erste und das zweite Umwandlungsmerkmal mindestens eines von einem Eingangsdynamikbereich und einer Ausgangsbitauflösung umfassen und das erste und das zweite Vorverarbeitungsmerkmal mindestens eines von einem Merkmal zum Filtern eines unnötigen Frequenzbandes und einem Merkmal zum Extrahieren von mindestens einem Satz von Vorverarbeitungsinformationen umfassen.

## Revendications

1. Processeur de signal biométrique parallèle (200), comprenant :
des ports (201-203) reliés à au moins un capteur conçu pour détecter au moins un signal biométrique ;
des amplificateurs (221-224) conçus pour amplifier le au moins un signal biométrique sur la base d'un attribut d'amplification ;
des convertisseurs (231-234) conçus pour convertir le signal amplifié en un signal converti sur la base d'un attribut de conversion ;
des préprocesseurs (241-244) conçus pour prétraiter le signal converti sur la base d'un attribut de prétraitement ; et
des extracteurs de caractéristiques (251-254) conçus pour extraire un ensemble d'informations biométriques à partir d'un signal de sortie des préprocesseurs ;
**le processeur de signal biométrique parallèle (200) comprenant en outre**
un dispositif de commande de commutation (260) conçu pour sélectionner un port cible parmi les ports (201-203), un amplificateur cible parmi les amplificateurs (221-224), un convertisseur cible parmi les convertisseurs (231-234), un préprocesseur cible parmi les préprocesseurs (241-244) et un extracteur de caractéristiques cibles parmi les extracteurs de caractéristiques (251-254) pour extraire des informations biométriques cibles ; et
des matrices de commutation (211-214) reliées parmi les ports (201-203), les amplificateurs (221-224), les convertisseurs (231-234), les préprocesseurs (241-244), et les extracteurs de caractéristiques (251-254),
une première matrice de commutation (211) reliant les ports (210-203) et les amplificateurs (221-224), une seconde matrice de commutation (212) reliant les amplificateurs (221-224) et les convertisseurs (231-234), une troisième matrice de commutation (213) reliant les convertisseurs (231-234) et les préprocesseurs (241-244) et une quatrième matrice de commutation (214) reliant les préprocesseurs (241-244) et les extracteurs de caractéristiques (251-254),
de sorte que le dispositif de commande de commutation (260) est conçu pour commander à la première à la quatrième matrice de commutation (211-214) d'effectuer un routage à travers le port cible, l'amplificateur cible, le convertisseur cible, le préprocesseur cible et l'extracteur de caractéristiques cibles du au moins un signal biométrique reçu au niveau du port cible.

2. Processeur selon la revendication 1, le dispositif de commande de commutation (260) étant conçu pour effectuer le re-routage à travers l'amplificateur cible, le convertisseur cible, le préprocesseur cible, et l'extracteur de caractéristiques cibles ; ou
le dispositif de commande de commutation (260) étant conçu pour effectuer le routage à travers l'amplificateur cible, le convertisseur cible, le préprocesseur cible, et l'extracteur de caractéristiques cibles sur la base d'un mode de fonctionnement.

3. Processeur selon la revendication 2, le mode de fonctionnement comprenant l'un d'un mode de fonctionnement basse puissance et d'un mode de fonctionnement de haute précision.

4. Processeur selon la revendication 1, les amplificateurs (221-224), les convertisseurs (231-234), les préprocesseurs (241-244), et les extracteurs de caractéristiques (251-254) étant reliés en parallèle, ou
les amplificateurs (221-224) possédant différents attributs amplifiés,
les convertisseurs (231-234) comprenant différents attributs de conversion,
les préprocesseurs (241-244) comprenant différents attributs de prétraitement, et
les extracteurs de caractéristiques (251-254) étant conçus pour extraire différents ensembles des informations biométriques.

5. Processeur selon la revendication 2,
le capteur comprenant au moins l'un d'un capteur d'électrode, d'un capteur photochimique, et d'un capteur photoélectrique.

6. Processeur selon la revendication 1, l'attribut amplifié comprenant au moins l'un d'une impédance d'entrée, d'une largeur de bande, et d'un gain d'amplification, ou
les amplificateurs (221-224) étant conçus pour ajuster l'attribut amplifié, ou
les amplificateurs (221-224) comprenant au moins l'un d'un amplificateur d'instrument, IA, d'un amplificateur de gain programmable, PGA, et d'un filtre passe-bande, BPF, ou
les convertisseurs (231-234) étant conçus pour convertir le signal amplifié en un signal numérique sur la base de l'attribut de conversion, ou
l'attribut de conversion comprenant au moins l'une d'une plage dynamique d'entrée et d'une résolution de bit de sortie, ou
l'attribut de prétraitement comprenant au moins l'un d'un attribut de filtrage d'une bande de fréquences non nécessaire du signal converti et d'un attribut d'extraction d'un ensemble d'informations de prétraitement à partir du signal converti, ledit au moins un ensemble d'informations de prétraitement comprenant au moins l'une d'informations sur un moment auquel le signal converti est acquis et d'informations sur une caractéristique de fréquence du signal converti.

7. Processeur selon la revendication 1, comprenant en outre :
un dispositif de commande de puissance (280) conçu pour réguler la puissance à fournir aux amplificateurs (221-224), aux convertisseurs (231-234), aux préprocesseurs (241-244), et aux extracteurs de caractéristiques (251-254), ou
un dispositif de commande de registre (270) conçu pour réguler les attributs détaillés des amplificateurs (221-224), des convertisseurs (231-234), des préprocesseurs (231-234), et des extracteurs de caractéristiques (251-254), ou
un transmetteur (620) conçu pour transmettre les informations biométriques à un dispositif externe.

8. Processeur selon la revendication 7, comprenant en outre :
une interface filaire (630) ou sans fil (640) reliée au dispositif externe,
le transmetteur (620) étant conçu pour transmettre les informations biométriques au dispositif externe en utilisant l'interface (630, 640) et/ou
l'interface filaire (630) reliée au dispositif externe comprenant au moins l'un d'un transmetteur récepteur asynchrone universel, UART, d'une interface périphérique en série, SPI, et d'un circuit inter-intégré, I2C, et
l'interface sans fil (640) reliée au dispositif externe comprenant au moins l'un d'un réseau de surface corporel, BAN, Bluetooth, ZigBee, et de communication en champ proche, NFC.

9. Processeur d'application (700), comprenant :
un cœur de processeur (710) conçu pour traiter des commandes et des données ; et
un processeur de signal biométrique parallèle (720) selon la revendication 1 conçu pour extraire un ensemble d'informations biométriques à partir d'un signal biométrique.

10. Support lisible par ordinateur ayant des instructions qui, lorsqu'exécutées par le processeur selon la revendication 1, amènent le processeur à effectuer les étapes de :
exécution d'un routage à travers les ports (201-203), les amplificateurs (221-224), les convertisseurs (231-234), les préprocesseurs (241-244), et les extracteurs de caractéristiques (251-254),
réception d'un signal biométrique à travers un port cible des ports (201-203) cibles ;
amplification de signal biométrique à travers un amplificateur cible des amplificateurs (221-224) ;
conversion de signal biométrique amplifié à travers un convertisseur cible des convertisseurs (231-234) ;
prétraitement du signal converti à travers un préprocesseur cible des préprocesseurs (241-244) ; et
extraction d'un ensemble d'informations biométriques à partir du signal prétraité à travers un extracteur de caractéristiques des extracteurs de caractéristiques (251-254) ;
l'exécution comprenant la sélection du port cible parmi les ports (201-203), l'amplificateur cible parmi les amplificateurs (221-224), le convertisseur cible parmi les convertisseurs (231-234), le préprocesseur cible parmi les préprocesseurs (241-244) et l'extracteur de caractéristiques cibles parmi les extracteurs de caractéristiques (251-254) pour extraire des informations biométriques cibles ; et
relier en outre les ports (201-203), les amplificateurs (221-224), les convertisseurs (231-234), les préprocesseurs (241-244), et les extracteurs de caractéristiques (251-254) à travers des matrices de commutation (211-214),
une première matrice de commutation (211) reliant les ports (210-203) et les amplificateurs (221-224), une seconde matrice de commutation (212) reliant les amplificateurs (221-224) et les convertisseurs (231-234), une troisième matrice de commutation (213) reliant les convertisseurs (231-234) et les préprocesseurs (241-244) et une quatrième matrice de commutation (214) reliant les préprocesseurs (241-244) et les extracteurs de caractéristiques (251-254),
pour commander à la première à la quatrième matrice de commutation (211-214) d'effectuer un routage à travers le port cible, l'amplificateur cible, le convertisseur cible, le préprocesseur cible et l'extracteur de caractéristiques cibles du signal biométrique reçu au niveau du port cible.

11. Procédé de fonctionnement d'un processeur de signal biométrique parallèle, comprenant les étapes de :
réception, par un dispositif de commande, d'un premier et d'un second signal, et traitement simultanément du premier et du second signal par le routage simultanément du premier signal à travers un premier trajet et du second signal à travers un second trajet,
conversion, à travers le premier trajet, par le dispositif de commande, du premier signal en un premier signal converti en utilisant un premier attribut de conversion, prétraitement du premier signal converti sur la base d'un premier attribut de prétraitement, et extraction de premières informations biométriques, et
conversion, à travers le second trajet, par le dispositif de commande, du second signal en un second signal converti en utilisant un second attribut de conversion, prétraitement du second signal converti sur la base d'un second attribut de prétraitement, et extraction de secondes informations biométriques ;
la réception comprenant la sélection d'un amplificateur cible, parmi des amplificateurs (221-224), correspondant à chacun du premier trajet et du second trajet, d'un convertisseur cible, parmi des convertisseurs (231-234), correspondant à chacun du premier trajet et du second trajet, d'un préprocesseur cible, parmi des préprocesseurs (241-244) correspondant à chacun du premier trajet et du second trajet, et d'un extracteur de caractéristiques cibles, parmi des extracteurs de caractéristiques (251-254), correspondant à chacun du premier trajet et du second trajet, et
liaison des amplificateurs (221-224), des convertisseurs (231-234), des préprocesseurs (241-244), et des extracteurs de caractéristiques (251-254) à travers des matrices de commutation (212-214) correspondant à chacun du premier trajet et du second trajet,
une première matrice de commutation (212) reliant les amplificateurs (221-224) et les convertisseurs (231-234), une seconde matrice de commutation (213) reliant les convertisseurs (231-234) et les préprocesseurs (241-244) et une troisième matrice de commutation (214) reliant les préprocesseurs (241-244) et les extracteurs de caractéristiques (251-254) correspondant à chacun du premier et du second trajet,
commandant ainsi à la première à la troisième matrice de commutation (212-214) d'effectuer un routage à travers l'amplificateur cible, le convertisseur cible, le préprocesseur cible et l'extracteur de caractéristiques cibles du premier et du second signal correspondant à chacun du premier et du second trajet.

12. Procédé selon la revendication 11, le dispositif de commande étant en outre conçu pour amplifier le premier signal en utilisant un premier attribut d'amplification et le second signal en utilisant un second attribut d'amplification avant la conversion du premier et du second signal, ou
comprenant en outre l'amplification, par le dispositif de commande, du premier et du second signal, la conversion, par des convertisseurs, du premier signal amplifié au premier signal converti et du second signal amplifié au second signal converti, le prétraitement, par des préprocesseurs, du premier signal converti et du second signal converti, et l'extraction, par des extracteurs de caractéristiques, des premières et secondes informations biométriques.

13. Procédé selon la revendication 11, le premier et le second attribut d'amplification comprenant au moins l'un d'une impédance d'entrée, d'une largeur de bande, et d'un gain d'amplification, le premier et le second attribut de conversion comprenant au moins l'une d'une plage dynamique d'entrée et d'une résolution de bit de sortie, et le premier et le second attribut de prétraitement comprenant au moins l'un d'un attribut de filtrage d'une bande de fréquences non nécessaire et d'un attribut d'extraction d'au moins un ensemble d'informations de prétraitement.
